(19) Europäisches Patentamt
European Patent Office
Office européen des brevets

(11) EP 2 700 942 B1

(12) **EUROPEAN PATENT SPECIFICATION**

(45) Date of publication and mention
of the grant of the patent:
**16.01.2019 Bulletin 2019/03**

(51) Int Cl.:
*G01N 33/36* (2006.01)    *G01N 15/08* (2006.01)
*G01N 5/02* (2006.01)

(21) Application number: **12774188.2**

(22) Date of filing: **17.04.2012**

(86) International application number:
**PCT/KR2012/002909**

(87) International publication number:
**WO 2012/144783 (26.10.2012 Gazette 2012/43)**

(54) **APPARATUS FOR MEASURING PERVIOUSNESS TO WATER VAPOR**

VORRICHTUNG ZUR MESSUNG EINER WASSERDAMPF-DURCHLÄSSIGKEIT

APPAREIL DE MESURE DE LA PERMÉABILITÉ À LA VAPEUR D'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.04.2011 KR 20110035895**

(43) Date of publication of application:
**26.02.2014 Bulletin 2014/09**

(73) Proprietor: **Korea Institute of Industrial
Technology
Choongcheongnam-do 331-825 (KR)**

(72) Inventors:
• **PARK, Jun Ho**
**Ansan Si**
**Gyeonggi-Do 426-171 (KR)**
• **NAM, Chang Woo**
**Seoul 158-754 (KR)**
• **CHA, Hee Cheol**
**Yongin Si**
**Gyeonggi-Do 446-908 (KR)**
• **SHIM, Huen Sup**
**Daegu 706-957 (KR)**
• **LIM, Jee Young**
**Jeonju Si**
**Jeollabuk-Do 561-231 (KR)**
• **LEE, Ji Yun**
**Incheon 450-734 (KR)**

(74) Representative: **Maikowski & Ninnemann
Patentanwälte Partnerschaft mbB
Postfach 15 09 20
10671 Berlin (DE)**

(56) References cited:
**EP-A1- 1 170 582      EP-A2- 0 344 465
JP-A- H08 323 132      JP-A- H10 244 123
JP-A- 2000 206 021     KR-B1- 100 752 776
US-A- 4 142 403        US-A- 4 312 219
US-A- 4 581 921**

## Description

[TECHNICAL FIELD]

The present invention relates to a moisture permeability measuring apparatus, and more particularly, to a moisture permeability measuring apparatus capable of measuring the moisture permeability of a functional fabric fixed in a moisture permeable cup in real time using an automated system according to a change in the temperature, humidity, and air current conditions in a chamber of constant temperature and humidity.

[BACKGROUND ART]

Recently, much attention has been paid to a multifunctional fiber, e.g., textile cloth that is highly breathable and that has a moisture-permeable and waterproof function capable of absorbing sweat therein and quickly emitting the sweat to the outside and preventing external moisture from penetrating thereinto. The textile cloth having the moisture-permeable and waterproof function has high moisture permeability which means that a degree to which heat and sweat of a human body generated in the textile cloth are emitted to the outside is high.

The moisture permeability means a property of cloth capable of emitting moisture and is thus used as an indicator for analyzing the performance of the cloth. In detail, the moisture permeability may be determined by measuring the weight (g) of vapor penetrating a fiber fabric for a predetermined time under specified temperature and moisture conditions. The higher the moisture permeability, the greater the moisture-permeable function of the fiber fabric is.

Representative examples of standard test methods that have been domestically used to measure moisture permeability in Korea include the calcium chloride method, the water method, and the potassium acetate method according to the KS K 0594 standards, and the hot plate assay according to the KS K ISO 11092 standards or the ISO 11092 standards.

These standard test methods used to measure moisture permeability will be described below.

First, the calcium chloride method will be described.

A constant temperature and humidity apparatus, a chemical scale having a sensitivity of 1 mg or more, a moisture permeable cup, and a moisture absorbent according to the KS M 8040 standards are needed to perform the calcium chloride method. Specifically, air satisfying specified temperature and humidity conditions circulates in the constant temperature and humidity apparatus at a wind speed of about 0.5 m/sec. Preferably, the moisture permeable cup does not allow vapor to pass therethrough and has an area that does not change even when the temperature thereof changes.

The calcium chloride method is performed as described below. Three sheets of circular samples each having a diameter of 7 cm are prepared to measure moisture permeability. Then, a specimen is formed by putting 33 g of a moisture absorbent into the moisture permeable cup preprocessed at about 40°C and controlling the distances between the moisture absorbent and the samples to be 3 mm. Then, the specimen is put into the constant temperature and humidity apparatus in which air having a temperature of $40\pm2$°C and relative humidity of $90\pm5\%$ circulates. After one hour passes, the specimen is removed from the constant temperature and humidity apparatus to measure the weight $a_1$ (mg) of the specimen. Then, after one hour passes again, the specimen is removed again from the constant temperature and humidity apparatus to measure the weight $a_2$ (mg) of the specimen.

The moisture permeability is calculated by Equation 1 below, and an average of moisture permeability is calculated by performing the process described above three times. In Equation 1, 'P' denotes the moisture permeability expressed in $g/(m^2h)$, '$(a_2-a_1)$' denotes a change in the masses of the samples per unit time, expressed in mg/h, and 'S' denotes a moisture permeable area expressed in $cm^2$.

[Equation 1]

$$P = \frac{10 \times (a_2 - a_1)}{S}$$

Here, the unit time is preferably converted into one hour or twenty-four hours.

Second, the water method will be described.

To perform the water method, a constant temperature and humidity apparatus, a chemical scale, and a moisture permeable cup are needed, like the calcium chloride method.

The water method is performed as described below. Three sheets of circular samples each having a diameter of 8 cm are prepared to measure moisture permeability. Then, the moisture permeable cup preprocessed at about 40°C and water of about 40°C, e.g., distilled water or ion-exchange water of about 40°C, are prepared. A specimen is formed by pouring 42 ml of the water into the moisture permeable cup and controlling the distances between the water and the samples to be 10 mm. Then, the specimen is put into the constant temperature and humidity apparatus in which air having a temperature of $40\pm2$°C and relative humidity of $90\pm5\%$ circulates. After one hour passes, the specimen is removed from the constant temperature and humidity apparatus to measure the weight $a_1$ (mg) of the specimen. Then, after one hour passes again, the specimen is removed again from the constant temperature and humidity apparatus to measure the weight $a_2$ (mg) of the specimen.

The moisture permeability is calculated by Equation 1 above, and an average of moisture permeability is calculated by performing the process described above three times.

Third, the potassium acetate method will be described.

To perform the potassium acetate method, a constant temperature and humidity apparatus, a chemical scale, a moisture permeable cup, a moisture absorbent according to the KS M 8318 standards, a sample support frame, an auxiliary moisture permeability measurement film, and a water tank are needed. Specifically, the constant temperature and humidity apparatus, the chemical scale, and the moisture permeable cup used in the potassium acetate method are the same as those used in the calcium chloride method. The sample support frame is preferably a cylindrical synthetic resin frame having an inner diameter of 80 mm, a height of 50 mm, and a thickness of 3 mm. The auxiliary moisture permeability measurement film is preferably a polytetrafluoroethylene (PTFE) film having a fine porous structure of a porosity of 80% and a thickness of about 25 $\mu$m. The water tank is preferably a structure capable of being put into the constant temperature and humidity apparatus and fixing the sample support frame.

The potassium acetate method is performed as described below. Three sheets of samples each having a size of 20×10 cm are prepared to measure moisture permeability, and the water tank that contains water of about 23°C is put into the constant temperature and humidity apparatus in which air of 30±2°C circulates. Specifically, the samples are fixed with a rubber band such that the backs thereof face the exterior of the sample support frame, and the sample support frame is put into the constant temperature and humidity apparatus. After fifteen minutes pass, a specimen is formed by pouring 2/3 of a potassium acetate solution of about 23°C into the moisture permeable cup and fixing the auxiliary moisture permeability measurement film with a rubber band. The weight $a_0$ (mg) of the specimen is measured in a state in which the auxiliary moisture permeability measurement film faces upward, and the specimen is put into the sample support frame fixed in the water tank. After fifteen minutes pass, the specimen is turned upside down and the weight $a_1$ (mg) of the specimen is measured.

The moisture permeability is calculated by Equation 2 below and an average of moisture permeability is calculated by performing the process described above three times. In Equation 2, 'P' denotes the moisture permeability expressed in g/(m²h), '($a_1$-$a_0$)' denotes a change in the masses of the samples per unit time, expressed in mg/min, and 'S' denotes a moisture permeable area expressed in cm².

[Equation 2]

$$P = \frac{40 \times (a_1 - a_0)}{S}$$

Fourth, the hot plate assay will be described.

To perform the hot plate assay, a measuring device capable of measuring temperature and controlling water supply, a thermal protector capable of performing temperature control, and an environment maintaining device capable of maintaining a measurement environment are needed.

The measuring device should be able to precisely maintain a measurement temperature Tm within a range of ±0.1 K using a temperature controller including a temperature sensor, and maintain heating power H within ±2% of a total range of use. The thermal protector should be able to maintain a temperature that is the same as the temperature of the measuring device within an error range of ±0.1 K or less using the temperature controller. The environment maintaining device is configured to accommodate the thermal protector therein, in which an internal temperature may vary within a range of ±0.1 K and humidity may vary within a range of ±3% R.H. Also, preferably, the environment maintaining device may circulate air at a height of 15 mm above from an upper surface of the measuring device at an average speed of 1 m/sec and allow the air to vary within a range of ±0.05 m/s.

With the hot plate assay, moisture permeability may be measured using a thermal resistor Rct or a moisture permeable resistor Ret. Preferably, when the moisture permeability is measured using the thermal resistor Rct, the measuring device may set a temperature to 35°C, a temperature of air to 20°C, relative humidity to 65% R.H., and the speed of the air to 1 m/s and then measure the moisture permeability. When the moisture permeability is measured using the moisture permeable resistor Ret, the measuring device may set a temperature of air to 35°C, relative humidity to 40% R.H., and the speed of the air to 1m/s, and then measure the moisture permeability.

In the hot plate assay, samples, the sizes of which are larger than 230×230 mm, are prepared to measure moisture permeability, the body of a tester, the measuring device, and the thermal protector are entirely covered with the samples, and the moisture permeability is then measured. A result of measuring the moisture permeability and a process of calculating the moisture permeability may be checked by a software means.

Even if the moisture permeability of the same fabric is measured using one of the above methods, i.e., a standard test method according to the standards, the moisture permeability may vary according to external measurement variables, e.g., the states of the constant temperature and humidity apparatus, the chemical scale, the tester, and calcium chloride, and water pressure.

That is, a conventional moisture permeability measuring apparatus measures moisture permeability in a state in which system automation is hardly established. Thus, not only does moisture permeability vary according to measurement conditions, but a variation in the moisture permeability is also high.

In particular, when moisture permeability is measured using the conventional moisture permeability measuring apparatus, a variation may occur in the measured moisture permeability according to test skills of a tester, e.g., different handlings with particular matters that are not specified in the standards. Also, a variation may occur in the measured moisture permeability due to imbalance of an air current in the constant temperature and humidity apparatus, a change in the location of the moisture permeable cup, and a change in a measurement time caused when a device is exposed to an external environment.

Furthermore, the higher the moisture permeability of a material, the greater a variation in the moisture permeability may be, and the moisture permeability of a material having high moisture permeable properties may be measured to be lower than that of a material having low moisture permeable properties.

Although a variation in a result of measuring moisture permeability may be slightly lower when the standard test methods according to the KS K ISO 11092 standards are used, a measurement time is long and measurement equipment is expensive to use in an enterprise field. Accordingly, the moisture permeability of a fabric is difficult to measure.

Thus, it is urgent to develop an automated moisture permeability measuring apparatus capable of being easily prepared in an enterprise field and easily measuring moisture permeability and enabling a measurement time to be shortened.

EP 0 344 465 A2 discloses a moisture content measuring system including a sample container for holding a sample, a weighing instrument having a turn-table adapted to rotate with the sample container placed thereon, an A/D converter responsive to a signal from the weighing instrument, a calculator responsive to a signal from the A/D converter for calculating a moisture content in the sample, an indicator responsive to a signal from the calculator and a microwave generating and irradiating apparatus for heating the sample. The system further comprises hot wind generator means for heating the sample at a low temperature, and dehumidified dry air circulating means for removing moisture in the sample.

US 4 312 219 A describes an apparatus that is capable of continuously measuring the weight loss of a light weight, porous sheet material during drying while in constant contact with a heated surface. The resulting data may be converted to drying rate curves suitable for analyzing the effects on drying rate of furnish additives and processing conditions. The testing device is particularly useful for evaluating grades of paper or paperboard having basis weights greater than 50 g/m2. The device includes an electrically heated arcuate plate maintained at a constant temperature. The paper sample to be tested is brought into good heat transfer contact with the plate by means of a tensioned fabric that exerts a normal load on the sample but does not interfere with mass transfer of the vapor away from the drying sample. An air ventilating system provides a low turbulence flow of air uniformly over the fabric covering the sample. The plate, sample and fabric are supported by a load sensor that measures changes in weight of the sample during drying to 0.01 g. A special electrical connector between the power source and the heating elements of the hot plate isolates the power source from interference with the weighing measurement. Current is supplied to mercury pots, independently supported of the hot plate. Probes mounted on the hot plate and connected to the plate heating elements partially submerge in the mercury pots, transmitting power to the plate.

US 4 142 403 A presents an apparatus for testing soils by measuring the rate of moisture loss from soil or sediment samples is disclosed, comprising a test chamber, a fan, a heater-thermostat, a chilled water humidity control device, an automatic balance scale connected to a continuous chart recorder, a shield around the balance pan, and a magnetic damper for the balance pan. The heater-thermostat maintains a constant temperature within the chamber, while the chilled water humidity control device maintains a constant relative humidity. The fan circulates air in the chamber, and the magnetic damper and shield around the balance pan allows the soil sample to be weighed without air currents causing the balance pan to vibrate. The balance scale continually weighs the sample, with the weight being recorded on the chart recorder, thus providing a record of the rate of moisture loss. A bell jar allows the testing to be done under vacuum conditions.

EP 1 170 582 A1 proposes a moisture permeability measuring method comprising the steps of measuring the weight of a specimen in constant temperature and humidity room without taking out the specimen from the constant temperature and humidity room to the atmosphere and without being affected by vibration and objectively evaluating the results of the weight measurement by detecting the state of the water vapor permeating a sample and the temperature of stored water.

[DISCLOSURE]

[TECHNICAL PROBLEM TO BE SOLVED]

To solve the above problems, the present invention is directed to a moisture permeability measuring apparatus capable of measuring the moisture permeability of a functional fabric fixed in a moisture permeable cup in real time using an automated system according to a change in the temperature, humidity, and air current conditions in a chamber of constant temperature and humidity.

[TECHNICAL SOLUTION]

According to the present invention, an apparatus for measuring moisture permeability includes: a chamber of constant temperature and humidity including a main door at a front portion thereof; a measuring module including at least one electronic scale configured to weigh a moisture permeable cup; and an external control apparatus configured to control the chamber and the measuring module and store measurement data from the at least one electronic scale, wherein the measuring module is disposed in the chamber and includes at least one fan disposed above the at least one electronic scale to circulate air in the measuring module.

It is preferable that the chamber includes a main fan configured to generate air flow from a lower portion thereof to an upper portion thereof.

The chamber may include a main fan configured to generate air flow from a lower portion thereof to an upper portion thereof, and the measuring module includes at least one fan disposed above the at least one electronic scale to circulate air in the measuring module.

It is preferable that the measuring module further includes: a cup fixing frame disposed on the at least one electronic scale to fix the moisture permeable cup; and an air current guiding frame configured to guide an air current generated by the at least one fan to a fabric surface of a test sample. The measuring module may further include an air current control plate disposed above the cup fixing frame with a gap therebetween, the air current control plate blocking a downward air current generated by the main fan. The measuring module may further include an antivibration frame disposed below the at least one electronic scale.

It is preferable that the moisture permeable cup includes: a sealing member disposed between the moisture permeable cup and an intermediate fixing plate; the intermediate fixing plate fixed by a fixing pin of the moisture permeable cup penetrating there through; and an upper fixing plate fastening the intermediate fixing plate, the test sample, and the moisture permeable cup to one another.

It is preferable that a vapor permeable area is defined in the intermediate fixing plate and the upper fixing plate.

It is preferable that the moisture permeable cup further includes one of a first saucer configured to adjust a height and a second saucer used for an inverse water method peformed by turning the moisture permeable cup upside down.

It is preferable that the external control apparatus includes a fan speed controller configured to control speeds of the main fan and the at least one fan. The external control apparatus may further include: a test time timer configured to control a total test time, a data storing interval and operations of the main fan and the at least one fan during a measurement; an electronic scale power supply configured to control an electric power to be supplied to the at least one electronic scale; and a system operating unit configured to perform operations of the external control apparatus, the operations including supplying the electric power, starting a test and terminating the test.

It is preferable that the test time timer includes: a total test time timer configured to set a total test time; a data transmission timer configured to set a data storing interval; and a fan stop time timer configured to stop the main fan and the at least one fan so as to stabilize an air current when storing the measurement data of the at least one electronic scale.

[ADVANTAGEOUS EFFECTS]

According to the present invention, the moisture permeability of a sample fabric may be measured using an automated system while changing temperature, humidity, and air current conditions. Also, the moisture permeability of the sample fabric may be measured at a predetermined location under the same conditions, e.g., the same temperature, humidity, and air current conditions.

Also, since the moisture permeability of the sample fabric is measured using an electronic scale only for a set time, the rate of measurement errors caused when a tester measures the moisture permeability by himself/herself may be low. A variation in the moisture permeability occurring during the set time may be checked in real time, thereby easily analyzing the moisture permeability of the sample fabric.

Also, since the moisture permeability measuring apparatus according to the present invention is capable of providing a simple test process and measurement method and is easy to attain proficiency in handling, the moisture permeability measuring apparatus is applicable to an enterprise field in which it is urgent to develop new fabrics. Also, the moisture permeability measuring apparatus according to the present invention is capable of measuring the moisture permeability of various types of fibers, e.g., a polymer film or a membrane.

[DESCRIPTION OF DRAWINGS]

     FIG. 1A is a front perspective view of a moisture permeability measuring apparatus according to the present invention, and FIG. 1B is a front perspective view of a measuring module according to the present invention.
     FIG. 2 illustrates the exterior of an external control apparatus according to the present invention.
     FIG. 3 is a side cross-sectional view of a chamber of constant temperature and humidity according to the present invention.
     FIG. 4A is a side cross-sectional view of a measuring module according to the present invention, FIG. 4B is a plan view of the measuring module according to the present invention, and FIG. 4C is a diagram illustrating a state in which a moisture permeable cup is disposed at a measuring module according to the present invention.
FIGS. 5A and 5B are cross-sectional views of the structures of moisture permeable cups according to the present invention.
FIGS. 6A and 6B are graphs showing results of tests conducted using a moisture permeability measuring apparatus according to the present invention.

[MODE OF THE INVENTION]

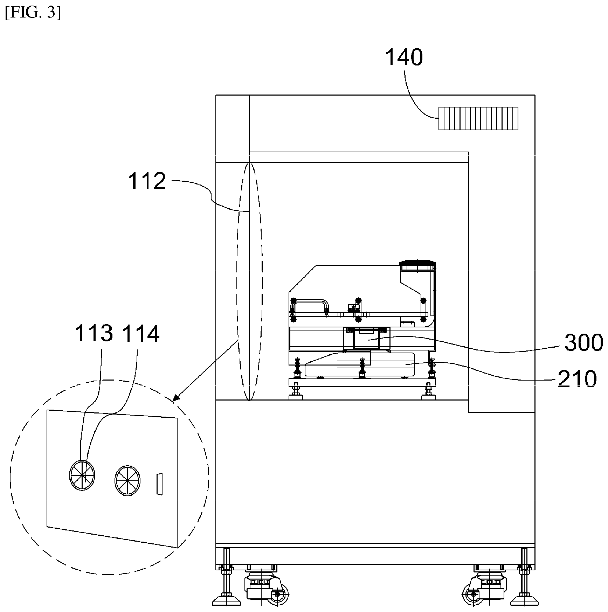

Hereinafter, the structure and effects of the present invention will be described in greater detail with reference to the accompanying drawings.

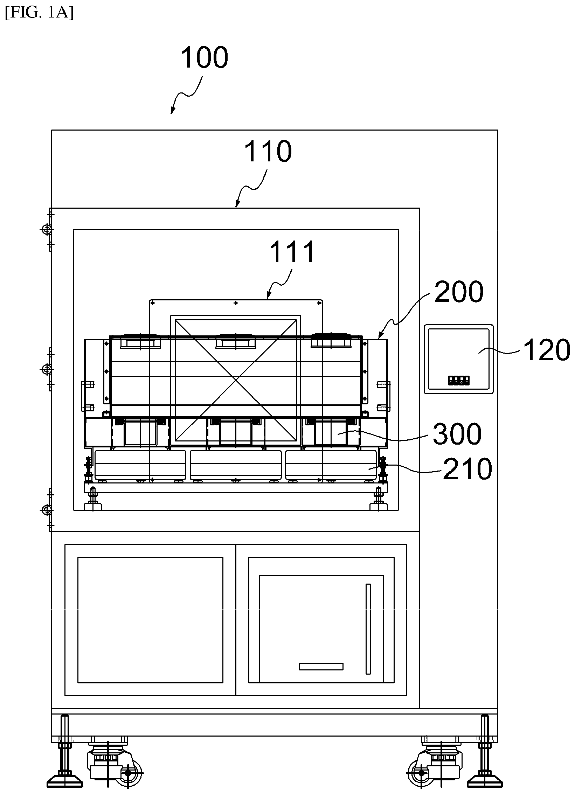

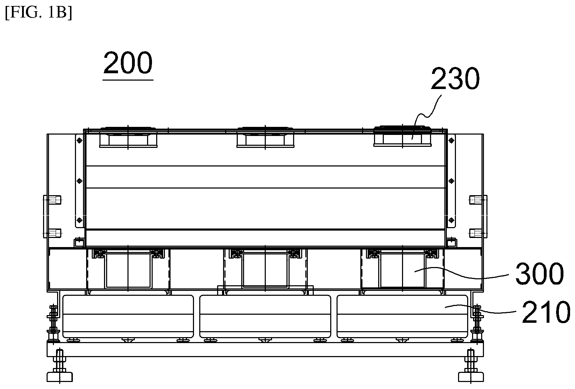

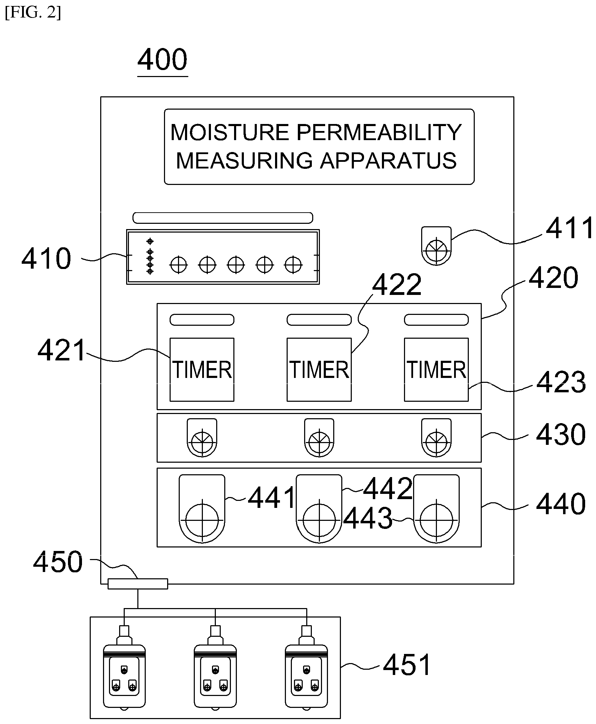

FIG. 1A is a front perspective view of moisture permeability measuring apparatus according to the present invention. FIG. 1B is a front perspective view of a measuring module 200 according to the present invention. FIG. 2 illustrates the exterior of an external control apparatus 400 according to the present invention.
Referring to FIGS. 1A and 2, the moisture permeability measuring apparatus according to the present invention includes a chamber 100 of constant temperature and humidity, the measuring module 200, and the external control apparatus 400.
The chamber 100 includes a main door 110 with a transparent observation window 111 at a front surface thereof. The chamber 100 further includes a main fan 140 illustrated in FIG. 3 configured to inhale internal air from a lower portion of the chamber 100 and emit the internal air to an upper portion of the chamber 100, a chamber controller 120 configured to set and control test conditions such as the temperature and humidity in the chamber 100, and a humidification water tank (not shown) configured to prepare a test environment.
The measuring module 200 is disposed in the chamber 100, and includes electronic scales 210 configured to measure the weight of at least one moisture permeable cup 300. The measuring module 200 further includes at least one small-sized fan 230 installed above the electronic scales 210 to circulate air in the measuring module 200.
The external control apparatus 400 controls the chamber 100 and the measuring module 200 and stores data measured by the electronic scales 210. Specifically, the external control apparatus 400 sets test conditions such as the fan speeds of the main fan 140 and the at least one small-sized fan 230 and a test time, and controls the test environment of the chamber 100 according to the set test conditions. The external control apparatus 400 stores test data received from the chamber 100 in a data storage apparatus 451 during a test.
As illustrated in FIG. 2, the external control apparatus 400 may include a fan speed controller 410 configured to control the fan speeds of the main fan 140 and the at least one small-sized fan 230.
Specifically, the external control apparatus 400 may control wind speeds of the main fan 140 installed in the chamber 100 and the at least one small-sized fan 230 using the fan speed controller 410, and control the operations of the fans 140 and 230 using a fan power switch 411.
The external control apparatus 400 may further include a test time timer 420 configured to control an entire test time, data storing intervals, and the operations of the fans 140 and 230; an electronic scale power supply 430 configured to control supplying of power to the electronic scales 210 so as to efficiently collect test data; and a system operating unit 440 configured to control an operation of the external control apparatus 400.
Specifically, the external control apparatus 400 may supply power to the electronic scales 210 via the electronic scale power supply 430 connected to each of the electronic scales 210, thereby preventing unnecessary test data and independently conducting tests on the electronic scales 210.
Preferably, the test time timer 420 may include a total test time timer 421 configured to set a total test time, a data transmission timer 422 configured to set data storing intervals, and a fan stop time timer 423 configured to stop the fans 140 and 230 so as to stabilize air currents during storing numerical values of the electronic scales 210.
Specifically, the external control apparatus 400 may set a time period for which the fans 140 and 230 are to be stopped using the fan stop time timer 423 so that the fans 140 and 230 may be temporarily stopped before the numerical values of the electronic scales 210 are stored. Thus, the external control apparatus 400 is capable of reading the numeral values of the electronic scales 210 in a state in which the air currents are stabilized, thereby increasing the precision of data.
The external control apparatus 400 may transmit data received from the electronic scales 210 to the data storage apparatus 451 via a data transmission cable 450 according to a set data transmission time.
FIG. 3 is a side cross-sectional view of the chamber 100 according to the present invention.
Referring to FIG. 3, an internal door 112 is installed at the chamber 100 to open or close an internal space of the chamber 100 that is isolated from the outside. The internal door 112 is preferably a double insulating glass door. Two working holes 114 each including an isolation film 113 that blocks an external environment may be formed in a center of the internal door 112 to minimize a change caused by the external environment even when additional work is required to be performed during a test.
The main fan 140 is disposed at the upper portion of the chamber 100 to generate an air flow from the lower portion of the chamber 100 to the upper portion of the chamber 100 using a vaporizer (not shown) and a heater (not shown), thereby enabling the temperature and humidity to be controlled in the chamber 100.

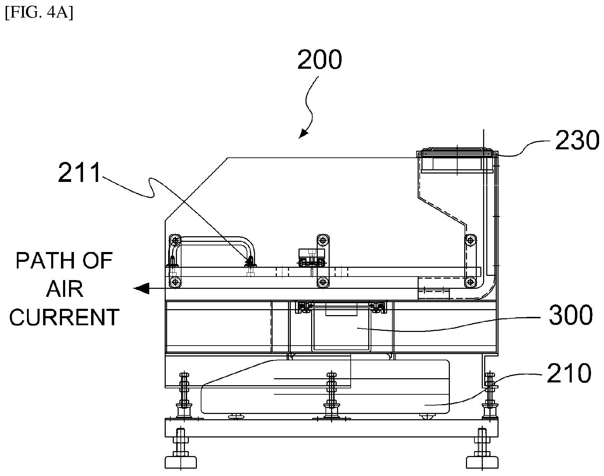

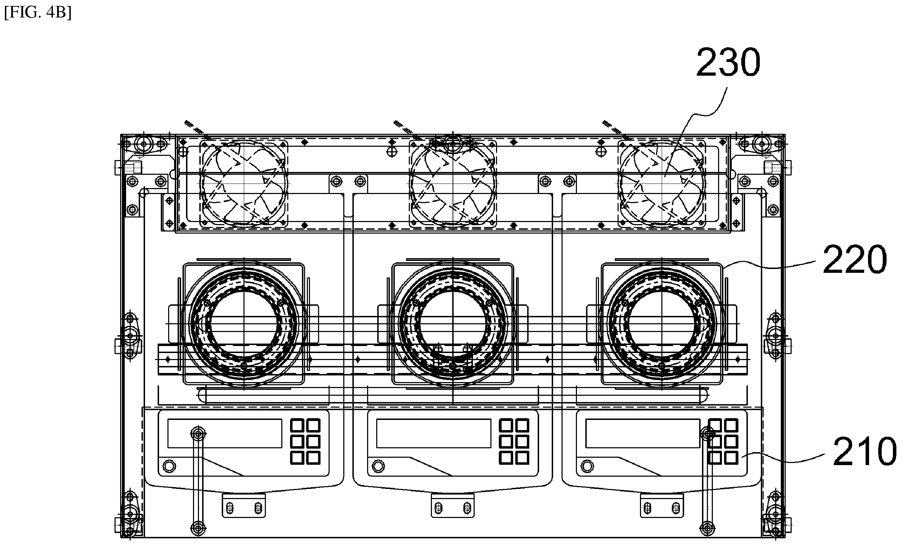

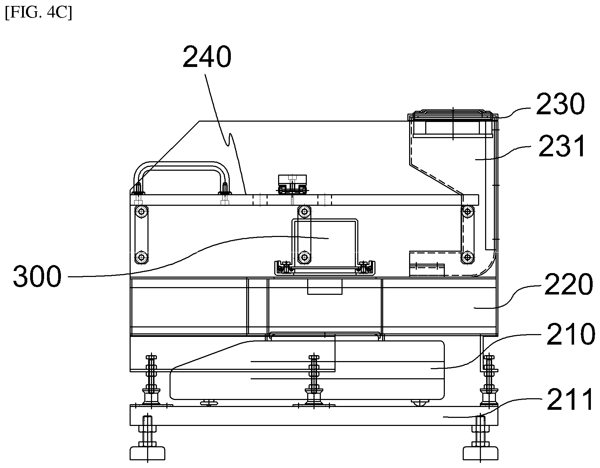

FIG. 4A is a side cross-sectional view of a measuring module 200 according to the present invention. FIG. 4B is a plan view of the measuring module 200 according to the present invention. FIG. 4C is a diagram illustrating a state in which the moisture permeable cup 300 is disposed at the measuring module 200 according to the present invention.

As illustrated in FIGS. 4A to 4C, the measuring module 200 includes at least one small-sized fan 230 installed above the electronic scales 210 to circulate air in the measuring module 200. The measuring module 200 may further include a cup fixing frame 220 disposed on the electronic scales 210 to fix the location of the moisture permeable cup 300, an air current guiding frame 231 configured to induce an air current generated by the at least one small-sized fan 230 to a fabric surface of a test sample, and an air current control plate 240 disposed above the cup fixing frame 220 to be apart from the cup fixing frame 220 and configured to control an air current formed by the at least one small-sized fan 230 to circulate in the chamber 100. Specifically, the air current control plate 240 blocks a downward air current formed by the main fan 140 so that the downward air current may not be supplied to the electronic scales 210, thereby preventing the weight of the moisture permeable cup 300 from being measured in an unstable state. Thus, the weight of the moisture permeable cup 300 may be precisely measured. The air current control plate 240 is preferably configured to be detachable and attachable.

Due to these elements of the measuring module 200, the air current circulating inside the chamber 100 may be moved into the moisture permeable cup 300. The measuring module 200 may further include a wind gauge 241, and measure the velocity of the air current circulating inside the chamber 100 using the wind gauge 241.

The measuring module 200 may include a plurality of electronic scales 210 to simultaneously measure moisture permeability. In this case, moisture permeability is preferably measured by installing small-sized fans 230, the number of which corresponds to the number of the plurality of electronic scales 210 above the electronic scales 210. For example, as illustrated in FIG. 4B, three electronic scales 210 may be installed to simultaneously perform a test on three samples, and three small-sized fans 230 may be installed above the three respective electronic scales 210 so that an air current may be simultaneously delivered to the three samples.

Also, a antivibration frame 211 may be disposed below the electronic scales 210 to stabilize a variation caused by vibration generated by the electronic scales 210 and to control vibration generated by the measuring module 200.

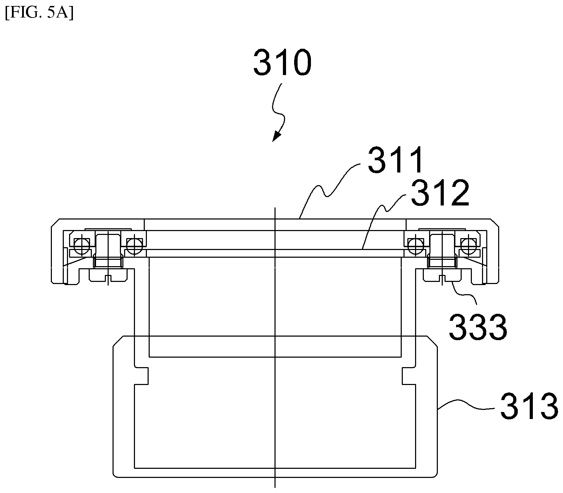

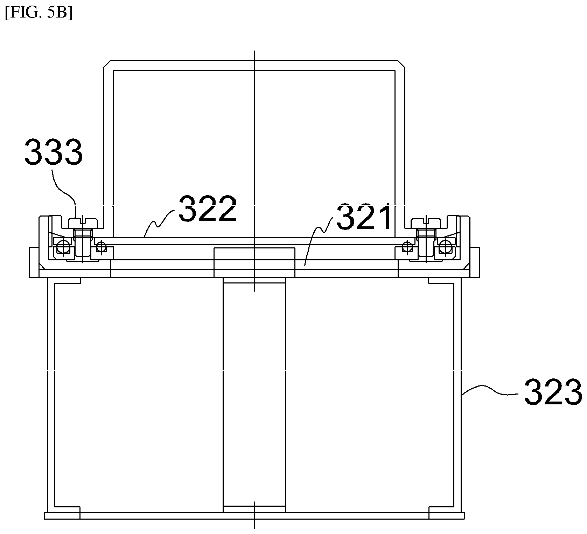

FIGS. 5A and 5B are cross-sectional views of the structures of moisture permeable cups according to the present invention.

A first moisture permeable cup 310 of FIG. 5A is a moisture permeable cup that may be used in the calcium chloride method, and a second moisture permeable cup 320 of FIG. 5B is a moisture permeable cup that may be used in the water method. The structures of the first moisture permeable cup 310 and the second moisture permeable cup 320 will be described in greater detail with reference to FIGS. 5A and 5B below.

Referring to FIG. 5A, the first moisture permeable cup 310 used in the calcium chloride method includes a first saucer 313 configured to equalize the height of the first moisture permeable cup 310 with that of the second moisture permeable cup 320 used in the water method when the calcium chloride method is performed using calcium chloride according to the moisture permeability measurement standards, an intermediate fixing plate 312 for fixing a sample fabric in the first moisture permeable cup 310, and an upper fixing plate 311 for firmly and closely adhering the intermediate fixing plate 312 and the sample fabric to each other.

The height of the first moisture permeable cup 310 used in the calcium chloride method is lower than that of the second moisture permeable cup 320 used in the water method according to the moisture permeability measurement standards. Thus, it is preferable that the first saucer 313 is used to adjust the height of the first moisture permeable cup 310 so that the air current generated by the at least one small-sized fan 230 can be supplied to the sample at an appropriate height.

Referring to FIG. 5B, the second moisture permeable cup 320 includes an intermediate fixing plate 322 for fixing a sample fabric in the second moisture permeable cup 320, an upper fixing plate 321 for firmly and closely adhering the intermediate fixing plate 322 and the sample fabric to each other, and a second saucer 323 for an inverse water method which is configured to assist in performing the inverse water method by turning the second moisture permeable cup 320 upside down.

Test areas in which the calcium chloride method and the water method are performed, respectively, may be an area including the intermediate fixing plate 312 and the upper fixing plate 311 for fixing the sample fabric and an area including the intermediate fixing plate 322 and the upper fixing plate 321 for fixing the sample fabric. Cylindrical vapor permeable areas corresponding to the test areas are preferably formed in the intermediate fixing plates 312 and 322 and the upper fixing plates 311 and 321, respectively.

The test area in the first moisture permeable cup 310 may have a diameter of 60 mm, and the test area in the second moisture permeable cup 320 may have a diameter of 70 mm. A ring-shaped packing (not shown) may be installed between the first moisture permeable cup 310 and the second moisture permeable cup 320 and between the intermediate fixing plates 312 and 322 to prevent vapor from leaking therefrom and flowing thereinto. Also, in the first moisture permeable cup 310 and the second moisture permeable cup 320, a fixing pin 333 may be installed to firmly fix the sample fabric onto the intermediate fixing plates 312 and 322 not to move from the intermediate fixing plates 312 and 322.

When moisture permeability is measured using a moisture permeability measuring apparatus according to the present invention, a range of controlling temperature and moisture in a constant temperature and humidity state and a range of controlling a test environment are as described below.

In a space in the chamber 100, a range of controlling temperature is +10 to 90°C (in the case of humidity conditions), a range of controlling humidity is 20 to 90% (when temperature is 20°C), and a range of controlling a wind speed is 0 to 15 m/sec. Preferably, ranges of controlling the temperature, humidity, and wind speed and a control time may be controlled by the chamber controller 120 and the external control apparatus 400.

A method of measuring moisture permeability using the moisture permeability measuring apparatus according to the present invention described above with reference to FIGS. 1A to 5B will now be described in detail.

First, a test method is selected to measure moisture permeability, and temperature and humidity in the chamber 100 are set according to test conditions. As an example, a method of measuring moisture permeability based on the calcium chloride method will be described below.

Second, the first moisture permeable cup 310 is inserted and fixed into the cup fixing frame 220 of the chamber 100, and the inside of the chamber 100 is stabilized to have the temperature and humidity corresponding to the test conditions.

After one hour passes, the main door 110 is opened and the first moisture permeable cup 310 is removed from the cup fixing frame 220 via the working hole 114 in the internal door 112. Then, calcium chloride according to the moisture permeability measurement standards is put into the first moisture permeable cup 310, and then a sample fabric that is to be tested is fixed with the intermediate fixing plate 312 and is firmly fastened to the intermediate fixing plate 312 with the upper fixing plate 311. That is, the sample fabric and the intermediate fixing plate 312 are fixed with the fixing pin 333 such that the fixing pin 333 passes through the sample fabric and the intermediate fixing plate 312, and are then firmly fastened to each other with the upper fixing plate 311, so that vapor may not flow into or leak from a space between the sample fabric and the first moisture permeable cup 310.

Then, the first moisture permeable cup 310 in which the sample fabric is fixed is placed on the cup fixing frame 220 in the chamber 100 via the working hole 114. In this case, the electronic scales 210 of the measuring module 200 should be set to zero.

Then, the speed of an air current in the chamber 100 is controlled with the fan speed controller 410 by supplying power to the external control apparatus 400, a test time is set by the test time timer 420, and measurement of moisture permeability is then started.

A user may freely set the test time using the test time timer 420. Specifically, the data transmission timer 422 and the fan stop time timer 423 may be controlled within a range of 0 to 9999 seconds. For example, a data transmission time may be set to be longer by about 5 seconds than a fan stop time so as to stably transmit data. However, the present invention is not limited thereto and the difference between the data transmission time and the fan stop time may be freely controlled when an air current is in a very stable state.

Then, the characteristics of the sample fabric may be determined by storing numerical values of the electronic scales 210 in the data storage apparatus 451 according to the set data transmission time and analyzing the numerical values stored in the data storage apparatus 451.

Preferably, a moisture permeability measuring apparatus according to the present invention is capable of measuring moisture permeability of various materials such as fabrics, knitted goods, vapor-permeable and waterproof fabrics, polymer films, and membranes.

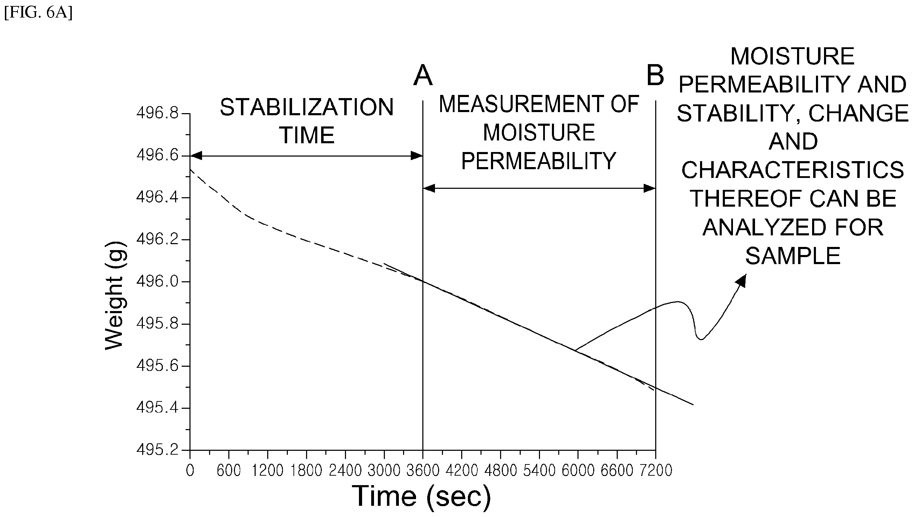

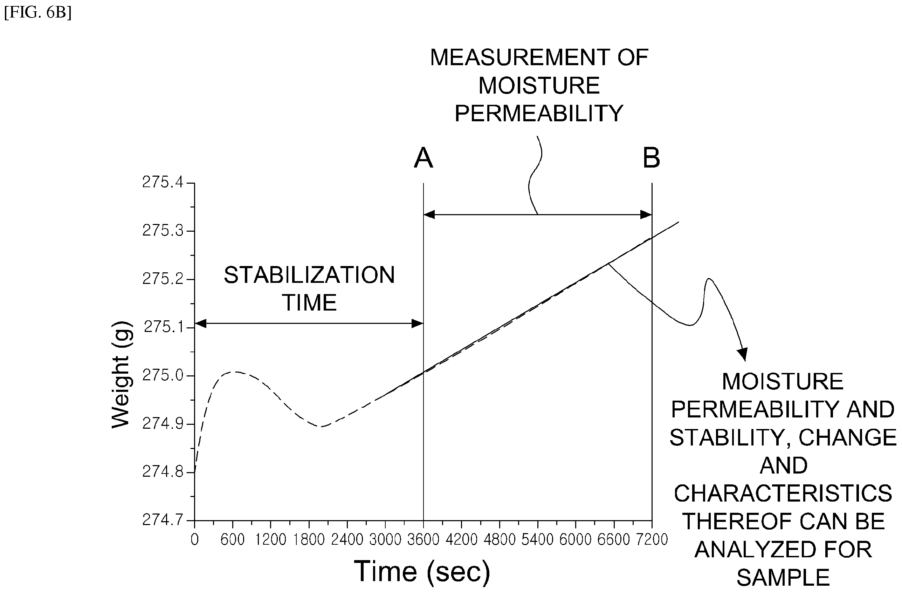

FIGS. 6A and 6B are graphs illustrating results of tests conducted using a moisture permeability measuring apparatus according to the present invention.

Conventionally, a result of measuring the weight of a moisture permeable cup at a point A and a point B by a tester is simply obtained. In contrast, with a moisture permeability measuring apparatus according to the present invention, it is possible to not only obtain a result of measuring the weight of a moisture permeable cup but also measure a variation in the weight of the moisture permeable cup in real time during a test. Thus, the tester is capable of analyzing the stability of and a change in the moisture permeability of each of sample fabrics and characteristics of the sample fabrics, based on the measurement result and the variation obtained during measurement of the moisture permeability.

That is, when the moisture permeability of a sample fabric is measured by applying the moisture permeability measuring apparatus according to the present invention to the water method, the stability of and a change in the moisture permeability of the sample fabric and characteristics of the sample fabric may be analyzed as illustrated in FIG. 6A. Also, when the moisture permeability of a sample fabric is measured by applying the moisture permeability measuring apparatus according to the present invention to the calcium chloride method, the stability of and a change in the moisture permeability of the sample fabric and characteristics of the sample fabric may be analyzed as illustrated in FIG. 6B.

Thus, a remarkably improved reliable measurement result and analysis result may be obtained when using the moisture permeability measuring apparatus according to the present invention compared to when a conventional moisture permeability measuring apparatus configured to obtain a result of measuring moisture permeability by simply checking a change in the weight of the moisture permeable cup is used.

**Claims**

1. An apparatus for measuring moisture permeability, the apparatus comprising:

   a chamber (100) of constant temperature and humidity including a main door (110) at a front portion thereof;
   a measuring module (200) including at least one electronic scale (210) configured to weigh a moisture permeable cup (300); and
   an external control apparatus (400) configured to control the chamber (100) and the measuring module (200) and store measurement data from the at least one electronic scale (210),
   **characterized in that** the measuring module (200) is disposed in the chamber (100) and comprises at least one fan (230) disposed above the at least one electronic scale (210) to circulate air in the measuring module (200).

2. The apparatus of claim 1, wherein the chamber (100) comprises a main fan (140) configured to generate air flow from a lower portion thereof to an upper portion thereof.

3. The apparatus of claim 1, wherein the measuring module (200) further comprises:

   a cup fixing frame (220) disposed on the at least one electronic scale (210) to fix the moisture permeable cup (300); and
   an air current guiding frame (231) configured to guide an air current generated by the at least one fan (230) to a fabric surface of a test sample.

4. The apparatus of claim 3, wherein the measuring module (200) further comprises an air current control plate (240) disposed above the cup fixing frame (220) with a gap therebetween, the air current control plate (240) blocking a downward air current generated by the main fan (140).

5. The apparatus of claim 4, wherein the measuring module (200) further comprises an antivibration frame (211) disposed below the at least one electronic scale (210).

6. The apparatus of claim 4, wherein the moisture permeable cup (300) comprises:

   a sealing member disposed between the moisture permeable cup (300) and an intermediate fixing plate (312);
   the intermediate fixing plate (312) fixed by a fixing pin of the moisture permeable cup (300) penetrating therethrough; and
an upper fixing plate (311) fastening the intermediate fixing plate (312), the test sample, and the moisture permeable cup (300) to one another.

7. The apparatus of claim 6, wherein a vapor permeable area is defined in the intermediate fixing plate (312) and the upper fixing plate (311).

8. The apparatus of claim 6, wherein the moisture permeable cup (300) further comprises one of a first saucer (313) configured to adjust a height and a second saucer (323) used for an inverse water method peformed by turning the moisture permeable cup (300) upside down.

9. The apparatus of one of claims 4 through 8, wherein the external control apparatus (400) comprises a fan speed controller (410) configured to control speeds of the main fan (140) and the at least one fan (230).

10. The apparatus of claim 9, wherein the external control apparatus (400) further comprises:

    a test time timer (420) configured to control a total test time, a data storing interval and operations of the main fan (140) and the at least one fan (230) during a measurement;
    an electronic scale power supply (430) configured to control an electric power to be supplied to the at least one electronic scale (210); and
    a system operating unit (440) configured to perform operations of the external control apparatus (400), the operations including supplying the electric power, starting a test and terminating the test.

11. The apparatus of claim 10, wherein the test time timer (420) comprises:

    a total test time timer (421) configured to set a total test time;
    a data transmission timer (422) configured to set a data storing interval; and
    a fan stop time timer (423) configured to stop the main fan (140) and the at least one fan (230) so as to stabilize an air current when storing the measurment data of the at least one electronic scale (210).

**Patentansprüche**

1. Vorrichtung zum Messen einer Feuchtigkeitsdurchlässigkeit, wobei die Vorrichtung Folgendes umfasst:

eine Kammer (100) mit konstanter Temperatur und Luftfeuchtigkeit, die eine Haupttür (110) bei einem Vorderteil davon enthält;

ein Messmodul (200), das mindestens eine elektronische Waage (210), die konfiguriert ist, eine feuchtigkeitsdurchlässige Schale (300) zu wiegen, enthält und

eine externe Steuervorrichtung (400), die konfiguriert ist, die Kammer (100) und das Messmodul (200) zu steuern und Messdaten von der mindestens einen elektronischen Waage (210) zu speichern,

**dadurch gekennzeichnet, dass** das Messmodul (200) in der Kammer (100) angeordnet ist und mindestens einen Ventilator (230) umfasst, der über der mindestens einen elektronischen Waage (210) angeordnet ist, um Luft im Messmodul (200) umzuwälzen.

2. Vorrichtung nach Anspruch 1, wobei die Kammer (100) einen Hauptventilator (140) umfasst, der konfiguriert ist, einen Luftstrom von einem unteren Teil davon zu einem oberen Teil davon zu erzeugen.

3. Vorrichtung nach Anspruch 1, wobei das Messmodul (200) ferner Folgendes umfasst:

einen Schalenbefestigungsrahmen (220), der auf der mindestens einen elektronischen Waage (210) angeordnet ist, um die feuchtigkeitsdurchlässige Schale (300) zu befestigen; und einen Luftstromleitrahmen (231), der konfiguriert ist, einen Luftstrom, der durch den mindestens einen Ventilator (230) erzeugt wird, zu einer Strukturoberfläche eines Prüfmusters zu leiten.

4. Vorrichtung nach Anspruch 3, wobei das Messmodul (200) ferner eine Luftstromsteuerplatte (240), die mit einem Spalt dazwischen über dem Schalenbefestigungsrahmen (220) angeordnet ist, umfasst, wobei die Luftstromsteuerplatte (240) einen Luftstrom nach unten, der durch den Hauptventilator (140) erzeugt wird, blockiert.

5. Vorrichtung nach Anspruch 4, wobei das Messmodul (200) ferner einen schwingungsdämpfenden Rahmen (211), der unter der mindestens einen elektronischen Waage (210) angeordnet ist, umfasst.

6. Vorrichtung nach Anspruch 4, wobei die feuchtigkeitsdurchlässige Schale (300) Folgendes umfasst:

ein Dichtungselement, das zwischen der feuchtigkeitsdurchlässigen Schale (300) und einer Zwischenbefestigungsplatte (312) angeordnet ist; wobei die Zwischenbefestigungsplatte (312) durch einen Befestigungsstift der feuchtigkeitsdurchlässigen Schale (300), der sie durchdringt, befestigt ist; und eine obere Befestigungsplatte (311), die die Zwischenbefestigungsplatte (312), das Prüfmuster und die feuchtigkeitsdurchlässige Schale (300) aneinander befestigt.

7. Vorrichtung nach Anspruch 6, wobei in der Zwischenbefestigungsplatte (312) und der oberen Befestigungsplatte (311) ein dampfdurchlässiger Bereich definiert ist.

8. Vorrichtung nach Anspruch 6, wobei die feuchtigkeitsdurchlässige Schale (300) ferner einen ersten Untersatz (313), der konfiguriert ist, eine Höhe anzupassen, und einen zweiten Untersatz (323), der für ein inverses Wasserverfahren verwendet wird, das durch auf den Kopf Stellen der feuchtigkeitsdurchlässigen Schale (300) durchgeführt wird, umfasst.

9. Vorrichtung nach einem der Ansprüche 4 bis 8, wobei die externe Steuervorrichtung (400) eine Ventilatordrehzahlsteuereinheit (410), die konfiguriert ist, Drehzahlen des Hauptventilators (140) und des mindestens einen Ventilators (230) zu steuern, umfasst.

10. Vorrichtung nach Anspruch 9, wobei die externe Steuervorrichtung (400) ferner Folgendes umfasst:

einen Prüfzeitzeitgeber (420), der konfiguriert ist, eine Gesamtprüfzeit, ein Datenspeicherintervall und den Betrieb des Hauptventilators (140) und des mindestens einen Ventilators (230) während einer Messung zu steuern; eine Leistungsversorgung (430) für elektronische Waagen, die konfiguriert ist, eine elektrische Leistung, die der mindestens einen elektronischen Waage (210) zugeführt werden soll, zu steuern; und eine Systembetriebseinheit (440), die konfiguriert ist, Vorgänge der externen Steuervorrichtung (400) durchzuführen, wobei die Vorgänge das Zuführen der elektrischen Leistung, das Starten einer Prüfung und das Beenden der Prüfung enthalten.

11. Vorrichtung nach Anspruch 10, wobei der Prüfzeitzeitgeber (420) Folgendes umfasst:

einen Gesamtprüfzeitzeitgeber (421), der konfiguriert ist, eine Gesamtprüfzeit einzustellen; einen Datenübertragungszeitgeber (422), der konfiguriert ist, ein Datenspeicherintervall einzustellen; und einen Ventilatorstoppzeitgeber (423), der konfiguriert ist, den Hauptventilator (140) und den mindestens einen Ventilator (230) anzuhalten, um einen Luftstrom zu stabilisieren, wenn die Messdaten der mindestens einen elektronischen Waage (210) gespeichert werden.

## Revendications

1. Appareil destiné à mesurer la perméabilité à l'humidité, l'appareil comportant :

   une chambre (100) de température et d'hygrométrie constantes comprenant une porte principale (110) à une partie avant de celle-ci ;
   un module (200) de mesure comprenant au moins une balance électronique (210) configuré pour peser un gobelet (300) perméable à l'humidité ; et
   un appareil (400) de commande externe configuré pour commander la chambre (100) et le module (200) de mesure et stocker des données de mesure provenant de la ou des balances électroniques (210),
   **caractérisé en ce que** le module (200) de mesure est disposé dans la chambre (100) et comporte au moins un ventilateur (230) disposé au-dessus de la ou des balances électroniques (210) pour faire circuler de l'air dans le module (200) de mesure.

2. Appareil selon la revendication 1, la chambre (100) comportant un ventilateur principal (140) configuré pour générer un écoulement d'air d'une partie inférieure de celle-ci à une partie supérieure de celle-ci.

3. Appareil selon la revendication 1, le module (200) de mesure comportant en outre :

   un châssis (220) de fixation de gobelet disposé sur la ou les balances électroniques (210) pour fixer le gobelet (300) perméable à l'humidité ; et
   un châssis (231) de guidage de courant d'air configuré pour guider un courant d'air généré par le ou les ventilateurs (230) jusqu'à une surface de tissu d'un échantillon de test.

4. Appareil selon la revendication 3, le module (200) de mesure comportant en outre une plaque (240) de régulation de courant d'air disposée au-dessus du châssis (220) de fixation de gobelet avec un écartement entre ceux-ci, la plaque (240) de régulation de courant d'air bloquant un courant d'air descendant généré par le ventilateur principal (140).

5. Appareil selon la revendication 4, le module (200) de mesure comportant en outre un châssis anti-vibrations (211) disposé au-dessous de la ou des balances électroniques (210).

6. Appareil selon la revendication 4, le gobelet (300) perméable à l'humidité comportant :

   un élément d'étanchéité disposé entre le gobelet (300) perméable à l'humidité et une plaque intermédiaire (312) de fixation ;
   la plaque intermédiaire (312) de fixation fixée par une goupille de fixation du gobelet (300) perméable à l'humidité passant à travers celle-ci ; et
   une plaque supérieure (311) de fixation assemblant entre eux la plaque intermédiaire (312) de fixation, l'échantillon de test et le gobelet (300) perméable à l'humidité.

7. Appareil selon la revendication 6, une zone perméable à la vapeur étant définie dans la plaque intermédiaire (312) de fixation et la plaque supérieure (311) de fixation.

8. Appareil selon la revendication 6, le gobelet (300) perméable à l'humidité comportant en outre soit une première soucoupe (313) configurée pour régler une hauteur, soit une deuxième soucoupe (323) utilisée pour un procédé inverse à l'eau réalisé en retournant le gobelet (300) perméable à l'humidité sens dessus dessous.

9. Appareil selon l'une des revendications 4 à 8, l'appareil (400) de commande externe comportant un régulateur (410) de vitesse de ventilateurs configuré pour réguler des vitesses du ventilateur principal (140) et du ou des ventilateurs (230).

10. Appareil selon la revendication 9, l'appareil (400) de commande externe comportant en outre :

    un temporisateur (420) de durée de test configuré pour commander une durée totale de test, un intervalle de stockage des données et le fonctionnement du ventilateur principal (140) et du ou des ventilateurs (230) pendant une mesure ;
    une alimentation (430) de balance électronique configurée pour réguler une puissance électrique à fournir à la ou aux balances électroniques (210) ; et
    une unité (440) d'exploitation de système configurée pour effectuer des opérations de l'appareil (400) de commande externe, les opérations comprenant la fourniture de la puissance électrique, le démarrage d'un test et l'arrêt du test.

11. Appareil selon la revendication 10, le temporisateur (420) de durée de test comportant :

    un temporisateur (421) de durée totale de test configuré pour régler une durée totale de test ;
    un temporisateur (422) de transmission de données configuré pour régler un intervalle de stockage des données ; et

un temporisateur (423) d'instant d'arrêt de ventilateurs configuré pour arrêter le ventilateur principal (140) et le ou les ventilateurs (230) de façon à stabiliser un courant d'air lors du stockage des données de mesure de la ou des balances électroniques (210).

[FIG. 1A]

[FIG. 1B]

[FIG. 2]

400

MOISTURE PERMEABILITY
MEASURING APPARATUS

410

411

422

420

421

TIMER    TIMER    TIMER

423

430

441    442
443

440

450

451

[FIG. 3]

[FIG. 4A]

[FIG. 4B]

[FIG. 4C]

240

230

231

300

220

210

211

[FIG. 5A]

310

311
312

333

313

[FIG. 5B]

[FIG. 6A]

EP 2 700 942 B1

[FIG. 6B]

MEASUREMENT OF
MOISTURE
PERMEABILITY

MOISTURE
PERMEABILITY AND
STABILITY, CHANGE
AND
CHARACTERISTICS
THEREOF CAN BE
ANALYZED FOR
SAMPLE

EP 2 700 942 B1

**EP 2 700 942 B1**

**REFERENCES CITED IN THE DESCRIPTION**

**Patent documents cited in the description**

- EP 0344465 A2
- US 4312219 A
- US 4142403 A
- EP 1170582 A1